# EUROPEAN PATENT SPECIFICATION

(11) **EP 0 446 873 B1**
(45) Date of publication and mention of the grant of the patent: **11.10.1995**
(21) Application number: 91103764.6
(22) Date of filing: 12.03.1991
(51) Int. Cl.: C07C 217/20, C07D 295/088, C07D 401/12, C07D 211/14, C07F 7/10, A01N 43/84

(54) **Amines showing fungicidal activity**
Amine mit fungizider Wirkung
Amines d'activité fongicide

(30) Priority: 13.03.1990 IT 1965390
(43) Date of publication of application: 18.09.1991
(73) Proprietor: MINISTERO DELL' UNIVERSITA' E DELLA RICERCA SCIENTIFICA E TECNOLOGICA, 00196 Roma (IT)
(72) Inventor: Camaggi, Giovanni, I-28100 Novara (IT); Filippini, Lucio, I-21047 Saronno, Varese (IT); Gusmeroli, Marilena, 20052 Monza, Milan (IT); Garavaglia, Carlo, 20012 Cuggiono Milan (IT); Mirenna, Luigi, 20139 Milan (IT)
(74) Representative: Weinhold, Peter, Dr.

(56) References cited:
- EP-A- 0 269 363
- DE-A- 1 670 635
- DE-A- 2 504 790
- DE-A- 3 410 070
- GB-A- 2 078 217
- PATENT ABSTRACTS OF JAPAN, unexamined applications, c field, vol. 4, 113, August 13, 1980, THE PATENT OFFICE JAPANESE GOVERNMENT page 121 C 21

## Description

The present invention relates to amines endowed with high antifungal activity, to a process for preparing them and to their use as fungicides in agriculture.

The DE-A-16 70 635 discloses o-(β-morpholinoethoxy)diphenyl ethers of formula 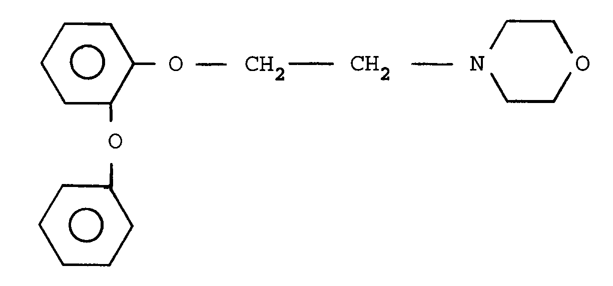
which are useful in treatment of hypertension.

The GB-A-2 078 217 describes compounds of general formula 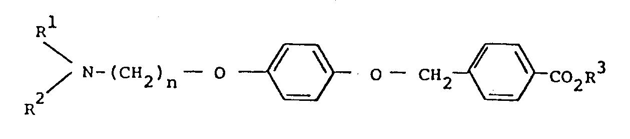
wherein R₁ may be joined to R₂ to form a pyrrolidinyl, piperidinyl or morpholinyl group; R₃ is a hydrogen atom or a C₁₋₆ alkyl group; and n is an integer from 2 to 6. These compounds are useful as antiviral agents. Similarly, JP-A-55-72149 (as cited in Patent Abstracts of Japan, vol. 4, 113, 1980, page 121 C 21) discloses aminoalkoxydiphenyl ethers of general formula 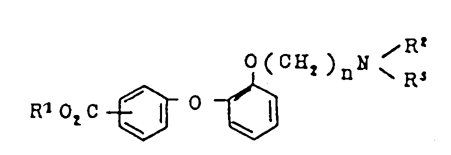
wherein R¹ is H or lower alkyl; R² and R³ are lower alkyl; and n is an integer of from 2 to 6. These compounds have antikematopexis and particularly blood platelet agglutination-suppression activity and are useful for prophylaxis and remedy for thrombosis.

The DE-A-25 04 790 discloses phenoxyalkylamines of general formula 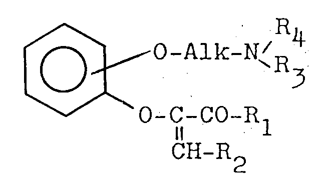
wherein R₁ and R₂ independently represent alkyl or aryl; Alk is an alkylene group; and the group NR₃R₄ being in ortho- or para-position is, for example, a piperidinyl or a morpholinyl group. Depending on the substituents these compounds have blood platelet agglutination-suppression activity or are useful as local anesthetics and for treatment of hypertension.

The DE-A-34 10 070 describes compounds such as 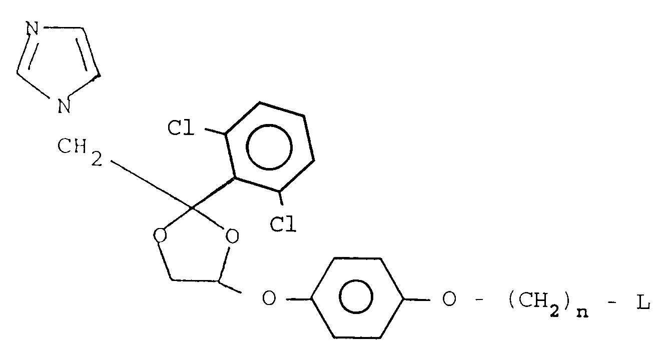
wherein n is an integer of from 2 to 10; and L may be, e.g., a piperidinyl group, a morpholinyl group, a thiomorpholinyl group or a substituted derivative thereof. These compounds are useful as chemotherapeutic agents in treatment of fungal infections.

The EP-A-0 269 363 discloses aminoalkylated hydroxy compounds endowed with fungicidal activity having the general formula 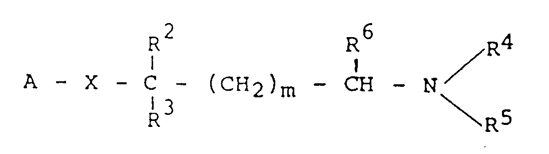
wherein A represents 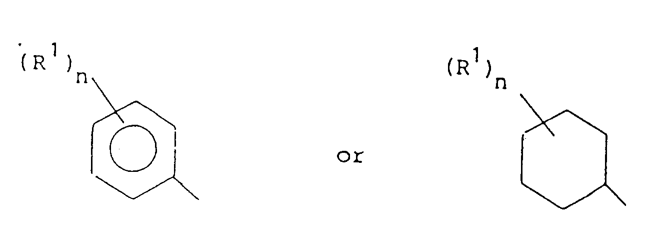
wherein (R¹)ₙ represents up to four substituents which are the same or different and selected from straight or branched chain, substituted or unsubstituted alkyl having up to 12 carbon atoms, aryl, aryloxy, alkoxy, arylalkyl, haloalkyl, dialkylamino, halogen, alkylthio and nitro, where two adjacent R¹-substituents may also together form a saturated or unsaturated ring, and n is 0, 1, 2, 3 or 4,
R² and R³ each independently represents hydrogen or straight or branched chain alkyl having up to 6 carbon atoms,
R⁴ and R⁵ each independently represents hydrogen, straight or branched chain or cyclic, substituted or unsubstituted alkyl having up to 12 carbon atoms, alkenyl, alkynyl or substituted or unsubstituted arylalkyl, or R⁴ and R⁵ when taken together with the nitrogen atom to which they are attached form a saturated or unsaturated, substituted, unsubstituted, aryl-fused or cycloalkyl-fused heterocyclic ring which may optionally contain one or more additional hetero atoms selected from O and N, R⁶ represents hydrogen or lower alkyl, X represents O or S, and
m is 0, 1 or 2 when A represents substituted or unsubstituted phenyl, and m is 0 or 1 when A represents substituted or unsubstituted cyclohexyl.

The object of the present invention are compounds of general formula (I): 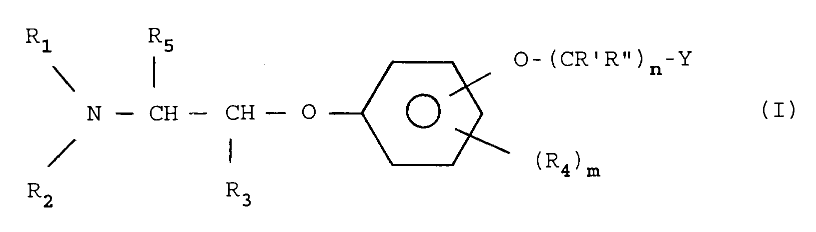
wherein:
- R₁ and R₂: together with the N atom to which they are bound represent a (C₃-C₈)-heterocyclic group or a (C₂-C₇)-heterocyclic group containing a second heteroatom selected from O and S, said heterocyclic groups optionally being substituted with at least one group selected from (C₁-C₄)-alkyl and halogen (e.g. F, Cl, Br, J, particularly Cl);
- R₃ and R₅: independently represent hydrogen or (C₁-C₃)-alkyl;
- R₄: represents halogen, (C₁-C₃)-alkyl or (C₁-C₃)-haloalkyl (e.g. methyl, ethyl and the corresponding groups having one or more halogen, particularly Cl substituents); in case m is ≧ 2 the groups R₄ being the same or different from each other;
- m: is an integer of from 0 to 4, particularly 0, 1 or 2;
- R' and R'': independently represent hydrogen or (C₁-C₃)-alkyl (e.g. methyl or ethyl);
- n: is an integer of from 0 to 3; and
- Y: represents a (C₃-C₆)-cycloalkyl group or a 5-or 6-membered heterocyclic group, said groups optionally being substituted with at least one group selected from (C₁-C₄)-alkyl (e.g. methyl or ethyl), (C₁-C₃)-alkoxy (e.g. methoxy or ethoxy), (C₁-C₃)-haloalkoxy, haloalkyl (see above), and halogen; or represents a group of formula 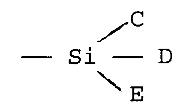 wherein C, D and E independently represent hydrogen, (C₁-C₄)-alkyl, (C₁-C₄)-haloalkyl, (C₁-C₄)-alkoxy, (C₁-C₄)-haloalkoxy, (C₆-C₁₀)-aryl or (C₆-C₁₀)-haloaryl (for specific examples of said groups see above),
as well as enantiomers, diastereoisomers, metal salts and complexes thereof.

The compounds of general formula (I) contain at least one asymmetric centre; the pure enantiomers or pure diastereoisomers, as well as mixtures thereof in any ratios, are comprised within the scope of the instant invention.

As used herein, "halogen" or "halo" denotes F, Cl, Br and I, if not specified otherwise.

Examples of aryl groups are phenyl, naphthyl and higher homologues.

Preferred examples of groups of formula 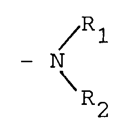
wherein N, R₁ and R₂ together represent a (C₃-C₈)- or (C₂-C₇)-heterocyclic group as defined above, are those groups which are derived from morpholine, 2,6-dimethyl-morpholine, piperidine, 2,6-dimethyl-piperidine and thiomorpholine. Said groups can also be substituted as defined above.

Preferred examples of 5- or 6-membered heterocyclic groups Y are those derived from pyridine, pyrimidine, thiophene, thiazole, oxazole and isoxazole, Said groups may optionally be substituted as defined above.

Among the groups Y which represent (C₃-C₆)-cycloalkyl groups, the cyclohexyl, cyclopropyl, cyclopentyl and cyclobutyl groups, optionally substituted as defined above, such as 1-methyl-2,2-dichloro-cyclopropyl, may be mentioned.

Among the silyl groups of formula SiCDE, trimethyl-silyl, tert.-butyl-dimethyl-silyl and dimethyl-phenyl-silyl groups are preferred examples.

Further objects of the present invention are:
- the salts of the compounds of general formula (I) deriving from an inorganic acid such as, e.g., a hydrogen halide (e.g. hydroiodic, hydrobromic or hydrochloric acid); sulfuric acid, nitric acid, thiocyanic acid and phosphoric acid; or from an organic acid such as, e.g., acetic acid, propanoic acid, ethane-dicarboxylic acid, propane-dicarboxylic acid, benzoic acid, salicyclic acid, saccharin, methane-sulfonic acid and 4-methyl-benzene-sulfonic acid;
- the metal complexes obtainable by a complex-forming reaction between the compounds of general formula (I) and an organic or inorganic metal salt such as, e.g., a halide, nitrate, sulfate, phosphate of, e.g., copper, manganese, zinc or iron. Said salts and complexes may be prepared according to well-known techniques.

The compounds of general formula (I) can be prepared by known, i.e., conventional methods.
A preferred method can be schematically represented as follows (for n = 1): 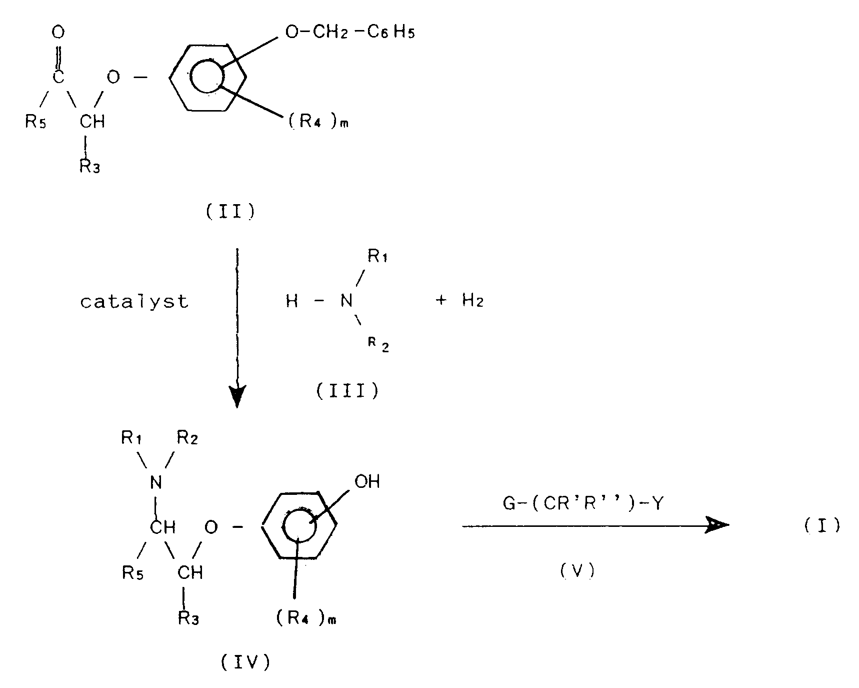
More specifically, the carbonyl compound (II) is hydrogenated in the presence of the amine (III) and of a conventional hydrogenation catalyst (Pd on charcoal, Raney-nickel etc.) to obtain the amine (IV). The hydrogen pressure preferably ranges from about 0.1 to about 1.1 MPa (about 1 to about 10 atmospheres), and the temperature preferably is in the range of from about 0°C to about 60°C (see J. March "Advanced Organic Chemistry", 2nd Edition, Int. St. Edition, page 819).

In the preparation scheme reported above, the symbols R₁, R₂, R₃, R₄, R₅, R', R'', Y and m have the previously defined meanings.

The compound (I) is obtained from the amine (IV) by reacting said amine with the compound (V), in which G denotes halogen (preferably Cl, Br or I), or an activated ester group (preferably methane-sulfonate or p-toluene-sulfonate). The reaction is carried out in the presence of an organic base (such as triethylamine and pyridine) or an inorganic base (such as sodium carbonate and sodium bicarbonate) in a dipolar protic solvent (such as water and ethanol) or aprotic solvent (such as N,N-dimethyl-formamide and N-methyl-pyrrolidone) at temperatures of from about 25°C to the boiling temperature of the solution (see J. March "Advanced Organic Chemistry", 2nd Edition, Int. St. Edition, page 357).

If desired, the metal salts and/or complexes of compounds (I) can then be prepared according to well-known techniques.

The carbonyl compounds (II) are commercially available or can be prepared according to known techniques.

The compounds (V) are also commercially available. When Y represents the group 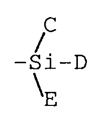
and at least one of the radicals C, D and E is (C₁-C₄)-perfluoroalkyl, compound (V) can be obtained according to methods known from the literature [JACS 73, 3518 (1951), Tetrahedron Letters 25, 2195 (1984)].

The amines (III) are products available on the market or they can easily be synthesized (see J. March "Advanced Organic Chemistry", 2nd Edition, Int. St. Edition, page 357).

The compounds of general formula (I) are endowed with a high activity as growth inhibitors of several species of pathogenic fungi which attack the cultivations of useful plants.

When they are applied to useful plants or parts thereof such as, e.g., leaves, the compounds of formula (I) show both a preventive and a curative activity, and are particularly effective in preventing diseases caused by pathogenic fungi, such as, e.g., those belonging to the genera Erysiphe and Helminthosporium.

Examples of plant diseases which can be controlled by the compounds according to the present invention are:
- Erysiphe graminis on cereals,
- Sphaeroteca fuliginea on Cucurbitaceae (e.g., cucumber),
- Puccinia on cereals,
- Septoria on cereals,
- Helminthosporium on cereals,
- Rhynchosporium on cereals,
- Podosphaera leucotricha on apple-trees,
- Uncinula necator on vines,
- Venturia inaequalis on apple-trees,
- Pyricularia oryzae on rice,
- Botrytis cinerea,
- Fusarium on cereals,
as well as other diseases.

For practical use in agriculture, it is often useful to have available antifungal compositions containing one or more components of formula (I) as active substances.

The application of these compositions may take place on any part of the plant, such as, e.g., leaves, stems, branches and roots, or on the seeds, before sowing, or also on the soil in which the plant grows. The compositions can be used in the form of dry powders, wettable powders, emulsifiable concentrates, pastes, granulates, solutions and suspensions; the selection of the particular type of composition will depend on the specific use.

The above compositions may be prepared in known manner, for example by diluting or dissolving the active substance with/in a solvent medium and/or a solid diluent, optionally in the presence of surfactants. As solid diluents, or supports, the following may, for instance, be used: silica, kaolin, bentonite, talc, fossil meal, dolomite, calcium carbonate, magnesium oxide, gypsum, clays, synthetic silicates, attapulgite and sepiolite.

The liquid diluents may be selected from water and various types of (organic) solvents such as, e.g., aromatic solvents (such as benzene, xylenes and mixtures of alkyl-benzenes), chloroaromatic compounds (such as chlorobenzene), paraffins (such as petroleum cuts), alcohols (such as methanol, propanol and butanol), amines, amides (e.g. dimethylformamide), ketones (e.g. cyclohexanone, acetophenone, isophorone and ethyl-amyl-ketone) and esters (e.g. isobutyl acetate).

As surfactants, the following may, for instance, be used: sodium, calcium or triethanolamine salts of alkyl-sulfates, alkyl-sulfonates, alkyl-aryl-sulfonates, polyethoxylated alkylphenols, adducts of ethylene oxide with fatty alcohols, polyethoxylated fatty acids, polyethoxylated sorbitol esters, polyethoxylated fats and ligno-sulfonates.

The compositions may also contain special additives for particular purposes, such as, e.g., binders such as gum arabic, polyvinyl alcohol and polyvinylpyrrolidone.

If desired, also other compatible active substances such as fungicides, plant growth regulators, herbicides, insecticides, fertilizers etc. can be added to the compositions according to the present invention.

The concentration of the active substance of formula (I) in the above compositions may vary as a function of the active compound, of the culture, of the pathogen, of environmental conditions and of the type of formulation adopted. Generally, however, the concentration of active agent will range from about 0.1% to about 95%, and preferably from about 0.5% to about 90% by weight.

The following examples are to illustrate the present invention without being a limitation thereof.

### EXAMPLE 1

### (a) Synthesis of 4-{3-[4-(trimethylsilylmethoxy)-phenyl]-2-methyl-3-oxa-propyl}-2,6-dimethyl-morpholine(Compound No. 1)

2.6 g of 4-[3-(4-(hydroxy-phenyl)-2-methyl-3-oxa-propyl]-2,6-dimethyl-morpholine are dissolved in 10 ml of dimethylformamide. To the resulting solution 5.5 g of anhydrous sodium carbonate are added, the resulting mixture is heated to 80°C under a nitrogen blanketing atmosphere, and is kept heated at this temperature for 30 minutes. Then 0.6 g of potassium iodide and 2.4 g of chloromethyl-trimethyl-silane are added and the heating of the mixture is continued for a further 4 hours. The reaction mixture is then quenched by pouring it into water and thereafter extracted with ethyl ether. The ether extract is then thoroughly dried and evaporated under reduced pressure. The resulting crude product is purified by chromatography on silica gel, with hexane/ethyl acetate = 9:1 as the eluent. Thus 2.3 g of compound 1 are obtained.

### Analysis:

NMR (60 MHz) in CDCl₃:
δ = 6.7 (4H, m)
4.3 (1H, m)
3.5 (4H, m)
0.8 - 2.8 (15H, m)
0.0 (9H, s)

### EXAMPLE 2

In similar manner, employing the corresponding starting materials, the following compounds 2 to 9 were synthesized.

### Compound No. 2

4-{3-[3-(2-(4,6-Dichloro)-pyridyloxy)-phenyl]-2-methyl-3-oxapropyl}-2,6-dimethyl-morpholine.
NMR (60 MHz) in CDCl₃:
δ = 8.0 (1H, d)
7.7 (1H, d)
6.9 (4H, m)
4.3 (1H, m)
3.6 (2H, m)
0.8 - 2.8 (15H, m)

### Compound No. 3

4-{3-[3-(2,2-Dichloro-1-methyl-cyclopropyl-methoxy)-phenyl]-2-methyl-3-oxapropyl}-2,6-dimethyl-morpholine.
NMR (60 MHz) in CDCl₃:
δ = 6.8 (4H, m)
4.3 (1H, m)
4.0 (2H, s)
3.6 (2H, m)
0.9 - 2.8 (20H, m)

### Compound No. 4

4-[3-(5-Trifluoromethylpyridyloxy-2-yl)phenyl-2-methyl-3-oxapropyl]-2,6-dimethyl-morpholine.
NMR (60 MHz) in CDCl₃:
δ = 8.4 (1H, m)
7.8 (1H, m)
7.4 - 6.6 (5H, m)
4.4 (1H, m)
3.5 (2H, m)
2.9 - 0.8 (15H, m)

### Compound No. 5

4-[3-(3-Chloro-5-trifluoromethylpyridyloxy-2-yl)-phenyl-2-methyl-3-oxapropyl]-2,6-dimethyl-morpholine.
NMR (60 MHz) in CDCl₃:
δ = 8.2 (1H, m)
7.8 (1H, m)
7.3 - 6.5 (4H, m)
4.4 (1H, m)
3.5 (2H, m)
2.9 - 0.8 (15H, m)

### Compound No. 6

4-[4-(Dimethylphenylsilylmethoxy)-phenyl-2-methyl-3-oxapropyl]-2,6-dimethyl-morpholine.
NMR (60 MHz) in CDCl₃:
δ = 7.8 - 7.2 (6H, m)
6.9 (3H, s)
4.4 (1H, m)
3.6 (4H, m + s)
2.8 - 0.8 (15H, m)
0.4 (6H, s)

### Compound No. 7

4-[3-(Dimethylphenylsilylmethoxy)-phenyl-2-methyl-3-oxapropyl]-2,6-dimethyl-morpholine.
NMR (60 MHz) in CDCl₃:
δ = 7.7 - 6.8 (7H, m)
6.4 (2H, m)
4.4 (1H, m)
3.6 (4H, m + s)
2.8 - 0.8 (15H, m)
0.3 (6H, s)

### Compound No. 8

4-{-(1-(Trimethylsilyl)ethoxy]phenyl-2-methyl-3-oxapropyl}-2,6-dimethyl-morpholine.
NMR (60 MHz) in CDCl₃:
δ = 7.1 (1H, m)
6.5 (3H, m)
4.5 (1H, m)
4.0 (1H, q)
3.6 (2H, m)
2.9 - 0.8 (18H, m)
0.0 (9H, s)

### Compound No. 9

4-{4-[1-(Trimethylsilyl)-ethoxy]phenyl-2-methyl-3-oxapropyl}-2,6-dimethyl-morpholine.
NMR (60 MHz) in CDCl₃:
δ = 6.8 (4H, m)
4.4 (1H, m)
3.9 (1H, q)
3.6 (2H, m)
2.9 - 0.9 (18H, m)
0.0 (9H, s)

### EXAMPLE 3

### Determination of the preventive fungicidal activity towards Helminthosporium teres

Both faces of leaves of plants of barley cv. Arna, grown in pots in a conditioned atmosphere, were sprayed with the tested products (Compounds Nos. 1 and 2) in water-acetone solution at 20% of acetone (volume/volume).

After 2 days in an atmosphere conditioned at 20°C and 70% R.H., both faces of the leaves of the plants were sprayed with an aqueous suspension of Helminthosporium teres (250,000 conidia/ml). After a further 24 hours in an atmosphere saturated with humidity, at 21°C, the plants were kept in a conditioned environment for fungus incubation.

At the end of said period (12 days), the severity of the infection was examined visually and rated according to a scale ranging from 100 (healthy plant) to 0 (completely infected plant).

The data obtained is summarized in Table 1.

**TABLE 1**

| COMPOUND NO. | DOSE (ppm) | HELMINTHOSPORIUM RATING |
|---|---|---|
| 1 | 500 | 100 |
| | 125 | 100 |
| 2 | 500 | 100 |
| | 125 | 100 |

### EXAMPLE 4

### Determination of the fungicidal activity towards corn oidium (Erysiphe graminis D.C.)

### Preventive Activity:

Both faces of leaves of plants of corn cv. Irnerio, grown in pots in a conditioned environment, were sprayed with the tested products (Compounds Nos. 1 and 2) in water-acetone solution at 20% of acetone (volume/volume).

After 1 day in an atmosphere conditioned at 20°C and 70% R.H., both faces of the leaves of the plants were sprayed with an aqueous suspension of Erysiphe graminis (200,000 conidia/ml). After a further 24 hours in an atmosphere saturated with humidity, at 21°C, the plants were kept in a conditioned atmosphere for fungus incubation.

At the end of said incubation period (12 days), the severity of the infection was examined visually and rated according to a scale ranging from 100 (healthy plant) down to 0 (completely infected plant).

### Curative Activity:

Both faces of leaves of plants of corn cv. Irnerio, grown in pots in a conditioned atmosphere, were sprayed with an aqueous suspension of Erysiphe graminis (200,000 conidia/ml). After 24 hours in an atmosphere saturated with humidity, at 21°C, the leaves were sprayed with the tested products (Compounds Nos. 1 and 2) in water-acetone solution at 20% of acetone (volume/volume).

At the end of the fungus incubation period (12 days), the severity of the infection was examined visually and rated according to a scale ranging from 100 (healthy plant) down to 0 (completely infected plant). The data obtained is summarized in Table 2.

**TABLE 2**

| COMPOUND NO. | DOSE (ppm) | ERYSIPHE RATING |
|---|---|---|
| 1 | 500 | 100 |
| | 250 | 100 |
| | 125 | 100 |
| 2 | 500 | 100 |
| | 250 | 100 |
| | 125 | 100 |

## Claims (Claims for the following Contracting State(s): AT, BE, CH, LI, DE, DK, FR, GB, GR, IT, NL, SE)

1. Compounds of general formula (I): 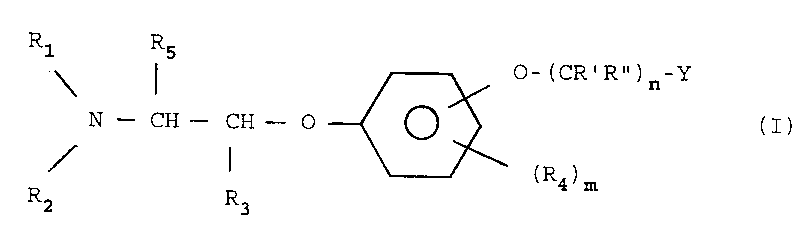 wherein:
R₁ and R₂ together with the N atom to which they are bound represent a (C₃-C₈)-heterocyclic group or a (C₂-C₇)-heterocyclic group containing a second heteroatom selected from O and S, said heterocyclic groups optionally being substituted with at least one group selected from (C₁-C₄)-alkyl and halogen;
R₃ and R₅ independently represent hydrogen or (C₁-C₃)-alkyl;
R₄ represents halogen, (C₁-C₃)-alkyl or (C₁-C₃)-haloalkyl
m is an integer of from 0 to 4;
R' and R'' independently represent hydrogen or (C₁-C₃)-alkyl;
n is an integer of from 0 to 3; and
Y represents a (C₃-C₆)-cycloalkyl group or a 5- or 6-membered heterocyclic group, said groups optionally being substituted with at least one group selected from (C₁-C₄)-alkyl, (C₁-C₃)-alkoxy, (C₁-C₃)-haloalkoxy, haloalkyl, and halogen; or represents a group of formula 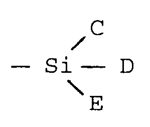 wherein C, D and E independently represent hydrogen, (C₁-C₄)-alkyl, (C₁-C₄)-haloalkyl, (C₁-C₄)-alkoxy, (C₁-C₄)-haloalkoxy, (C₆-C₁₀)-aryl or (C₆-C₁₀)-haloaryl,
as well as enantiomers, diastereoisomers, metal salts and complexes thereof.

2. Compounds according to claim 1, in which said (C₃-C₈)-heterocyclic group or (C₂-C₇)-heterocyclic group NR₁R₂ is selected from morpholinyl, piperidinyl and thiomorpholinyl, said groups optionally being substituted with at least one group selected from (C₁-C₄)-alkyl and halogen.

3. Compounds according to claim 1, in which said (C₃-C₈)-heterocyclic group or (C₂-C₇)-heterocyclic group NR₁R₂ is selected from morpholinyl, 2,6-dimethyl-morpholinyl, piperidinyl and 2,6-dimethyl-piperidinyl.

4. Compound according to any one of claims 1 to 3, in which Y is selected from pyridyl, pyrimidinyl, thienyl, thiazolyl, oxazolyl, isoxazolyl, cyclopropyl, cyclobutyl, cyclopentyl, cyclohexyl, 1-methyl-2,2-dichloro-cyclopropyl, trimethylsilyl, tert. -butyl-dimethylsilyl and dimethylphenylsilyl.

5. Compound according to claim 1, i.e., 4-{3-[4-(trimethylsilylmethoxy)-phenyl]-2-methyl-3-oxapropyl}-2,6-dimethyl-morpholine; 4-{3-[3-(2-(4,6-dichloro)-pyridyloxy)-phenyl]-2-methyl-3-oxapropyl}-2,6-dimethyl-morpholine; 4-{3-[3-(2,2-dichloro-1-methyl-cyclopropylmethoxy)phenyl]-2-methyl-3-oxapropyl}-2,6-dimethyl-morpholine; 4-[3-(5-trifluoromethylpyridyloxy-2-yl)phenyl-2-methyl-3-oxapropyl]-2,6-dimethyl-morpholine; 4-[3-(3-chloro-5-trifluoromethylpyridyloxy-2-yl)phenyl-2-methyl-3-oxapropyl]-2,6-dimethyl-morpholine; 4-[4-(dimethylphenylsilylmethoxy)-phenyl-2-methyl-3-oxapropyl]-2,6-dimethyl-morpholine; 4-[3-(dimethylphenylsilylmethoxy)-phenyl-2-methyl-3-oxapropyl]-2,6-dimethyl-morpholine; 4-{3-[1-(trimethylsilyl)ethoxy]phenyl-2-methyl-3-oxapropyl}-2,6-dimethyl-morpholine; 4-{4-[1-(trimethylsilyl)-ethoxy]phenyl-2-methyl-3-oxapropyl}-2,6-dimethyl-morpholine; or 4-{3-[1-(trimethylsilyl)ethoxy]phenyl-2-methyl-3-oxapropyl}-2,6-dimethyl-morpholine.

6. Process for preparing the compounds defined in any one of the preceding claims, comprising the hydrogenation of a carbonyl compound of general formula (II): 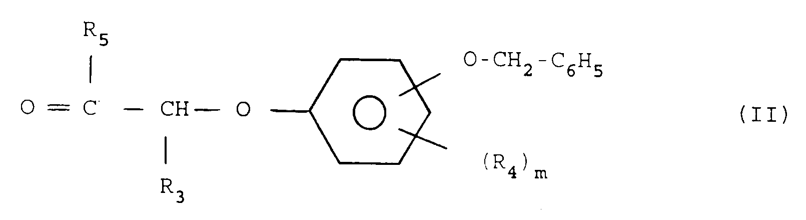 wherein R₃, R₄, R₅ and m are as defined in claim 1; after the addition of an amine of general formula (III): 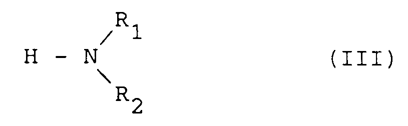 wherein R₁ and R₂ are as defined in claim 1; in the presence of a hydrogenation catalyst and at a temperature of from about 0 to about 60°C and with a hydrogen pressure of from about 0.1 to about 1.1 MPa (about 1 to about 10 atmospheres), resulting in the formation of the amine of general formula (IV): 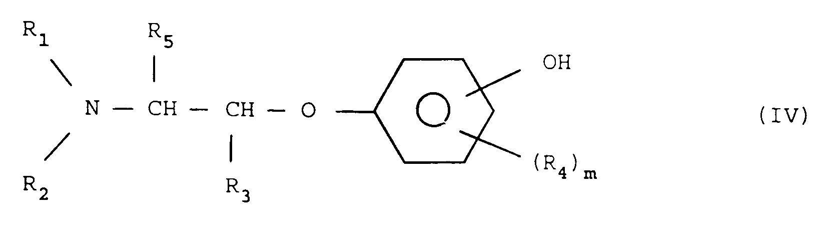 which, after reaction with the compound of general formula (V):
G-(CR'R'')ₙ-Y (V)
wherein n, R', R'' and Y are as defined in claim 1 and G = Cl, Br, I, a methane-sulfonate ester or p-toluene-sulfonate ester group; in the presence of a base in a dipolar protic or aprotic solvent and at a temperature of from about 25°C to the boiling point of the reaction mixture, yields the compound of the general formula (I); and, optionally, the conversion of the obtained compound into its corresponding salt or metal complex by conventional methods.

7. Fungicidal compositions, containing at least one of the compounds according to any one of claims 1 to 5 along with a solvent and/or diluent.

8. Use of the compounds according to any one of claims 1 to 5, as agents for inhibiting the growth of pathogenic fungi in cultivations of useful plants, in particular of fungi belonging to the genera Erysiphe and Helminthosporium.

## Claims (Claims for the following Contracting State(s): ES)

1. Process for preparing the compounds of general formula (I): 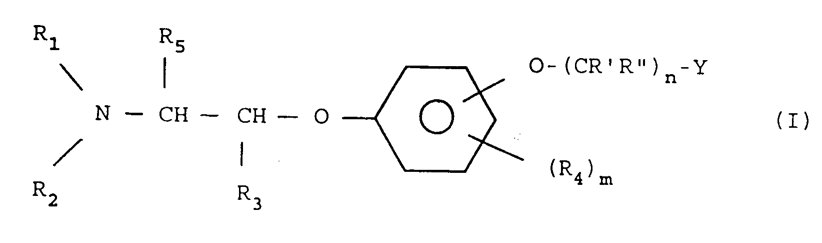 wherein:
R₁ and R₂ together with the N atom to which they are bound represent a (C₃-C₈)-heterocyclic group or a (C₂-C₇)-heterocyclic group containing a second heteroatom selected from O and S, said heterocyclic groups optionally being substituted with at least one group selected from (C₁-C₄)-alkyl and halogen;
R₃ and R₅ independently represent hydrogen or (C₁-C₃)-alkyl;
R₄ represents halogen, (C₁-C₃)-alkyl or (C₁-C₃)-haloalkyl
m is an integer of from 0 to 4;
R' and R'' independently represent hydrogen or (C₁-C₃)-alkyl;
n is an integer of from 0 to 3; and
Y represents a (C₃-C₆)-cycloalkyl group or a 5-or 6-membered heterocyclic group, said groups optionally being substituted with at least one group selected from (C₁-C₄)-alkyl, (C₁-C₃)-alkoxy, (C₁-C₃)-haloalkoxy, haloalkyl, and halogen; or represents a group of formula 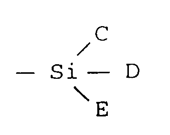 wherein C, D and E independently represent hydrogen, (C₁-C₄)-alkyl, (C₁-C₄)-haloalkyl, (C₁-C₄)-alkoxy, (C₁-C₄)-haloalkoxy, (C₆-C₁₀)-aryl or (C₆-C₁₀)-haloaryl,
as well as enantiomers, diastereoisomers, metal salts and complexes thereof, comprising the hydrogenation of a carbonyl compound of general formula (II): 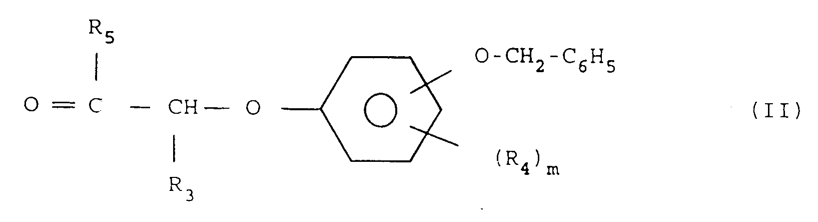 wherein R₃, R₄, R₅ and m are as defined above; after the addition of an amine of general formula (III): 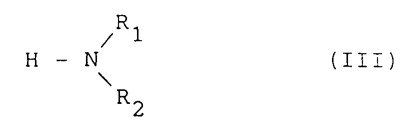 wherein R₁ and R₂ are as defined above; in the presence of a hydrogenation catalyst and at a temperature of from about 0 to about 60°C and with a hydrogen pressure of from about 0.1 to about 1.1 MPa (about 1 to about 10 atmospheres), resulting in the formation of the amine of general formula (IV): 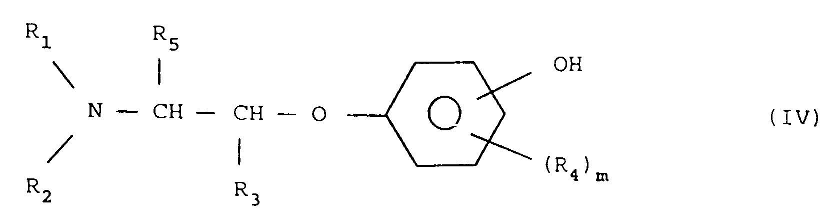 which, after reaction with the compound of general formula (V):
G-(CR'R'')ₙ-Y (V)
wherein n, R', R'' and Y are as defined in claim 1 and G = Cl, Br, I, a methane-sulfonate ester or p-toluene-sulfonate ester group; in the presence of a base in a dipolar protic or aprotic solvent and at a temperature of from about 25°C to the boiling point of the reaction mixture, yields the compound of the general formula (I); and, optionally, the conversion of the obtained compound into its corresponding salt or metal complex by conventional methods.

2. Process according to claim 1 for preparing the compounds of general formula (I), in which said (C₃-C₈)-heterocyclic group or (C₂-C₇)-heterocyclic group NR₁R₂ is selected from morpholinyl, piperidinyl and thiomorpholinyl, said groups optionally being substituted with at least one group selected from (C₁-C₄)-alkyl and halogen.

3. Process according to claim 1 for preparing the compounds of general formula (I), in which said (C₃-C₈)-heterocyclic group or (C₂-C₇)-heterocyclic group NR₁R₂ is selected from morpholinyl, 2,6-dimethyl-morpholinyl, piperidinyl and 2,6-dimethyl-piperidinyl.

4. Process according to any of claims 1 to 3 for preparing the compounds of general formula (I), in which Y is selected from pyridyl, pyrimidinyl, thienyl, thiazolyl, oxazolyl, isoxazolyl, cyclopropyl, cyclobutyl, cyclopentyl, cyclohexyl, 1-methyl-2,2-dichloro-cyclopropyl, trimethylsilyl, tert. -butyl-dimethylsilyl and dimethylphenylsilyl.

5. Process according to claim 1 for preparing the compounds of general formula (I), i.e., 4-(3-[4-(trimethylsilylmethoxy)-phenyl]-2-methyl-3-oxapropyl}-2,6-dimethyl-morpholine; 4-{3-[3-(2-(4,6-dichloro)-pyridyloxy)-phenyl]-2-methyl-3-oxapropyl}-2,6-dimethyl-morpholine; 4-{3-[3-(2,2-dichloro-1-methyl-cyclopropylmethoxy)phenyl]-2-methyl-3-oxapropyl}-2,6-dimethyl-morpholine; 4-[3-(5-trifluoromethylpyridyloxy-2-yl)phenyl-2-methyl-3-oxapropyl]-2,6-dimethyl-morpholine; 4-[3-(3-chloro-5-trifluoromethylpyridyloxy-2-yl)phenyl-2-methyl-3-oxapropyl]-2,6-dimethyl-morpholine; 4-[4-(dimethylphenylsilylmethoxy)-phenyl-2-methyl-3-oxapropyl]-2,6-dimethyl-morpholine; 4-[3-(dimethylphenylsilylmethoxy)-phenyl-2-methyl-3-oxapropyl]-2,6-dimethyl-morpholine; 4-{3-[1-(trimethylsilyl)ethoxy]phenyl-2-methyl-3-oxapropyl}-2,6-dimethyl-morpholine; 4-{4-[1-(trimethylsilyl)-ethoxy]phenyl-2-methyl-3-oxapropyl}-2,6-dimethyl-morpholine; or 4-{3-[1-(trimethylsilyl)ethoxy]phenyl-2-methyl-3-oxapropyl}-2,6-dimethyl-morpholine.

6. Fungicidal compositions, containing at least one of the compounds of the general formula (I) prepared according to any one of claims 1 to 5, along with a solvent and/or diluent.

7. Use of the compounds of the general formula (I) prepared according to anyone of the claims 1 to 5, as agents for inhibiting the growth of pathogenic fungi in cultivations of useful plants, in particular of fungi belonging to the genera Erysiphe and Helminthosporium.

## Patentansprüche (Patentansprüche für folgende(n) Vertragsstaat(en): AT, BE, CH, LI, DE, DK, FR, GB, GR, IT, NL, SE)

1. Verbindungen der allgemeinen Formel (I): 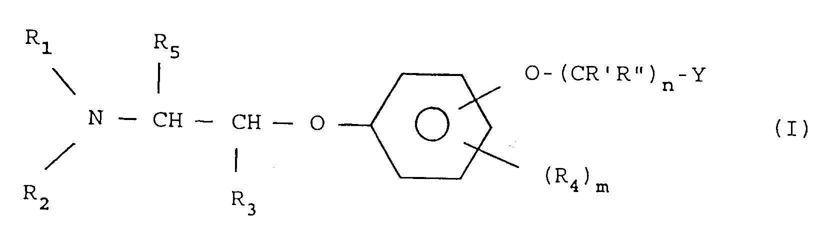 worin:
R₁ und R₂ zusammen mit dem N-Atom, an das sie gebunden sind, für eine heterocyclische (C₃-C₈)-Gruppe oder eine heterocyclische (C₂-C₇)-Gruppe, welche ein zweites, aus O und S ausgewähltes Heteroatom enthält, stehen, wobei diese heterocyclischen Gruppen gegebenenfalls mit mindestens einer Gruppe, ausgewählt aus (C₁-C₄)-Alkyl und Halogen, substituiert sind;
R₃ und R₅ unabhängig voneinander für Wasserstoff oder (C₁-C₃)-Alkyl stehen;
R₄ für Halogen, (C₁-C₃)-Alkyl oder (C₁-C₃)-Halogenalkyl steht;
m eine ganze Zahl von 0 bis 4 ist;
R' and R'' unabhängig voneinander für Wasserstoff oder (C₁-C₃)-Alkyl stehen;
n eine ganze Zahl von 0 bis 3 ist; und
Y für eine (C₃-C₆)-Cycloalkylgruppe oder für eine fünf-oder sechsgliedrige heterocyclische Gruppe steht, wobei diese Gruppen gegebenenfalls mit mindestens einer Gruppe, ausgewählt aus (C₁-C₄)-Alkyl, (C₁-C₃)-Alkoxy, (C₁-C₃)-Halogenalkoxy, Halogenalkyl und Halogen, substituiert sind; oder für eine Gruppe der Formel 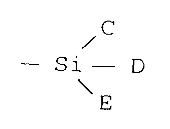 steht, worin C, D und E unabhängig voneinander für Wasserstoff, (C₁-C₄)-Alkyl, (C₁-C₄)-Halogenalkyl, (C₁-C₄)-Alkoxy, (C₁-C₄)-Halogenalkoxy, (C₆-C₁₀)-Aryl oder (C₆-C₁₀)-Halogenaryl stehen,
sowie Enantiomere, Diastereomere, Metallsalze und Komplexe davon.

2. Verbindungen gemäß Anspruch 1, bei denen die heterocyclische (C₃-C₈)-Gruppe oder die heterocyclische (C₂-C₇)-Gruppe NR₁R₂ ausgewählt ist aus Morpholinyl, Piperidinyl und Thiomorpholinyl, wobei diese Gruppen gegebenenfalls mit mindestens einer Gruppe, ausgewählt aus (C₁-c₄)-Alkyl und Halogen, substituiert sind.

3. Verbindungen gemäß Anspruch 1, bei denen die heterocyclische (C₃-C₈)-Gruppe oder die heterocyclische (C₂-C₇)-Gruppe NR₁R₂ ausgewählt ist aus Morpholinyl, 2,6-Dimethyl-morpholinyl, Piperidinyl und 2,6-Dimethyl-piperidinyl.

4. Verbindung gemäß irgendeinem der Ansprüche 1 bis 3, bei der Y ausgewählt ist aus Pyridyl, Pyrimidinyl, Thienyl, Thiazolyl, Oxazolyl, Isoxazolyl, Cyclopropyl, Cyclobutyl, Cyclopentyl, Cyclohexyl, 1-Methyl-2,2-dichloro-cyclopropyl, Trimethylsilyl, tert. -Butyl-dimethylsilyl und Dimethylphenylsilyl.

5. Verbindung gemäß Anspruch 1, d.h., 4-{3-[4-(Trimethylsilylmethoxy)-phenyl]-2-methyl-3-oxapropyl}-2,6-dimethylmorpholin; 4-{3-[3-(2-(4,6-Dichloro)-pyridyloxy)-phenyl]-2-methyl-3-oxapropyl}-2,6-dimethyl-morpholin; 4-{3-[3-(2,2-Dichloro-1-methyl-cyclopropylmethoxy)phenyl]-2-metyhl-3-oxapropyl}-2,6-dimethyl-morpholin; 4-[3-(5-Trifluoromethylpyridyloxy-2-yl)phenyl-2-methyl-3-oxapropyl]-2,6-dimethyl-morpholin; 4-[3-(3-Chloro-5-trifluoromethylpyridyloxy-2-yl)phenyl-2-methyl-3-oxapropyl]-2,6-dimethyl-morpholin; 4-[4-(Dimethylphenylsilylmethoxy)-phenyl-2-methyl-3-oxapropyl]-2,6-dimethyl-morpholin; 4-[3-(Dimethylphenylsilylmethoxy)-phenyl-2-methyl-3-oxapropyl]-2,6-dimethyl-morpholin; 4-{3-[1-(Trimethylsilyl)ethoxy]phenyl-2-methyl-3-oxapropyl}-2,6-dimethyl-morpholin; 4-{4-[1-(Trimethylsilyl)-ethoxy]phehyl-2-methyl-3-oxapropyl}-2,6-dimethyl-morpholin; oder 4-{3-[1-(Trimethylsilyl)ethoxy}]phenyl-2-methyl-3-oxapropyl}-2,6-dimethyl- morpholin.

6. Verfahren zur Herstellung der Verbindungen gemäß der Definitionen irgendeines der vorausgehenden Ansprüche, umfassend die Hydrierung einer Carbonylverbindung der allgemeinen Formel (II): 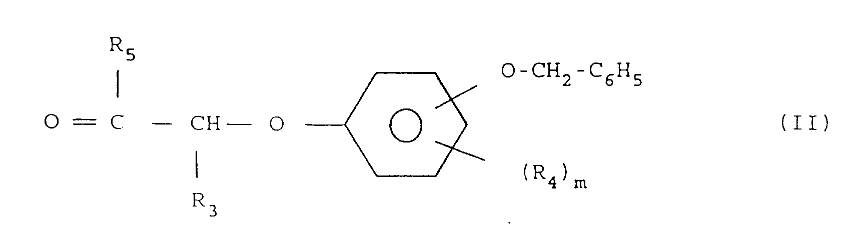 worin R₃, R₄, R₅ und m wie in Anspruch 1 definiert sind; nach der Zugabe eines Amins der allgemeinen Formel (III): 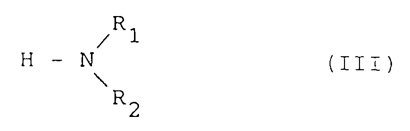 worin R₁ und R₂ wie in Anspruch 1 definiert sind; in der Gegenwart eines Hydrierungskatalysators bei einer Temperatur von etwa 0°C bis etwa 60°C und einem Wasserstoffdruck von etwa 0,1 bis etwa 1,1 MPa (etwa 1 bis etwa 10 atm), wodurch sich das Amin der allgemeinen Formel (IV): 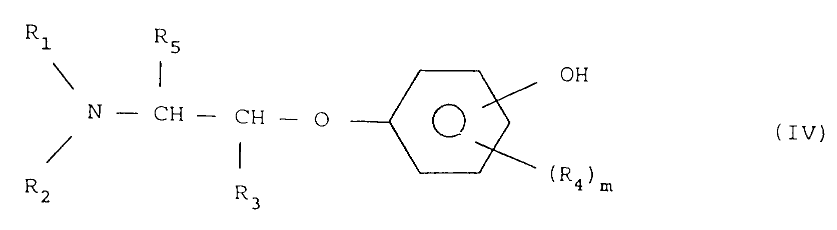 bildet, welches bei einer Umsetzung mit der Verbindung der allgemeinen Formel (V):
G-(CR'R'')ₙ-Y (V)
worin n, R', R'' und Y wie in Anspruch 1 definiert sind und G = Cl, Br, I, eine Methansulfonatester-Gruppe oder eine p-Toluolsulfonatester-Gruppe ist, in einem dipolaren protischen oder aprotischen Lösungsmittel in Gegenwart einer Base bei einer Temperatur zwischen etwa 25°C und dem Siedepunkt der Reaktionsmischung eine Verbindung der allgemeinen Formel (I) ergibt; sowie gegebenenfalls die Umsetzung der erhaltenen Verbindung mittels bekannter Verfahren in - das entsprechende Salz oder den Metallkomplex.

7. Fungizide Zusammensetzungen, welche mindestens eine der Verbindungen gemäß irgendeinem der Ansprüche 1 bis 5 zusammen mit einem Lösungsmittel und/oder einem Verdünnungsmittel enthalten.

8. Verwendung der Verbindungen gemäß irgendeinem der Ansprüche 1 bis 5 als Wirkstoffe zur Hemmung des Wachstums von Pilzen, welche für nützliche Kulturpflanzen pathogen sind, insbesondere von Pilzen, welche zur Art Erysiphe und Helminthosporium gehören.

## Patentansprüche (Patentansprüche für folgende(n) Vertragsstaat(en): ES)

1. Verfahren zur Herstellung von Verbindungen der allgemeinen Formel (I): 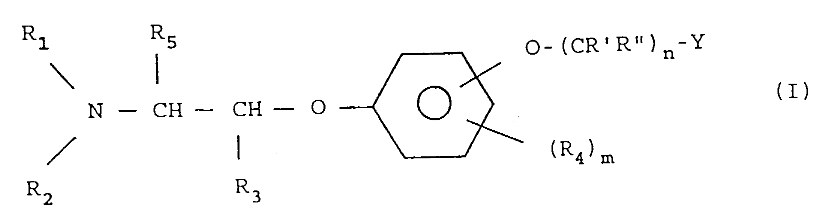 worin:
R₁ und R₂ zusammen mit dem N-Atom, an das sie gebunden sind, für eine heterocyclische (C₃-C₈)-Gruppe oder eine heterocyclische (C₂-C₇)-Gruppe, welche ein zweites, aus O und S ausgewähltes Heteroatom enthält, stehen, wobei diese heterocyclischen Gruppen gegebenenfalls mit mindestens einer Gruppe, ausgewählt aus (C₁-C₄)-Alkyl und Halogen, substituiert sind;
R₃ und R₅ unabhängig voneinander für Wasserstoff oder (C₁-C₃)-Alkyl stehen;
R₄ für Halogen, (C₁-C₃)-Alkyl oder (C₁-C₃)-Halogenalkyl steht;
m eine ganze Zahl von 0 bis 4 ist;
R' and R'' unabhängig voneinander für Wasserstoff oder (C₁-C₃)-Alkyl stehen;
n eine ganze Zahl von 0 bis 3 ist; und
Y für eine (C₃-C₆)-Cycloalkylgruppe oder für eine fünf- oder sechsgliedrige heterocyclische Gruppe steht, wobei diese Gruppen gegebenenfalls mit mindestens einer Gruppe, ausgewählt aus (C₁-C₄)-Alkyl, (C₁-C₃)-Alkoxy, (C₁-C₃)-Halogenalkoxy, Halogenalkyl und Halogen, substituiert sind; oder für eine Gruppe der Formel 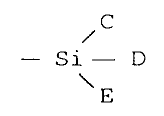 steht, worin C, D und E unabhängig voneinander für Wasserstoff, (C₁-C₄)-Alkyl, (C₁-C₄)-Halogenalkyl, (C₁-C₄)-Alkoxy, (C₁-C₄)-Halogenalkoxy, (C₆-C₁₀)-Aryl oder (C₆-C₁₀)-Halogenaryl stehen,
sowie Enantiomere, Diastereomere, Metallsalze und Komplexe davon, umfassend die Hydrierung einer Carbonylverbindung der allgemeinen Formel (II): 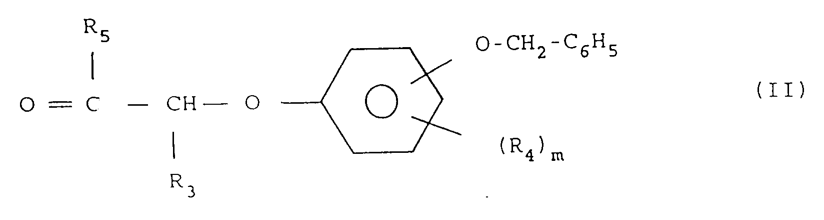 worin R₃, R₄, R₅ und m wie in Anspruch 1 definiert sind; nach der Zugabe eines Amins der allgemeinen Formel (III): 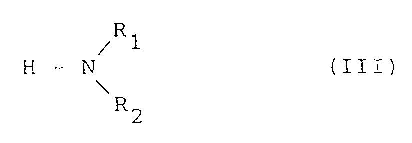 worin R₁ und R₂ die oben genannten Bedeutungen haben; in der Gegenwart eines Hydrierungskatalysators bei einer Temperatur von etwa 0°C bis etwa 60°C und einem Wasserstoffdruck von etwa 0,1 bis etwa 1,1 MPa (etwa 1 bis etwa 10 atm), wodurch sich das Amin der allgemeinen Formel (IV): 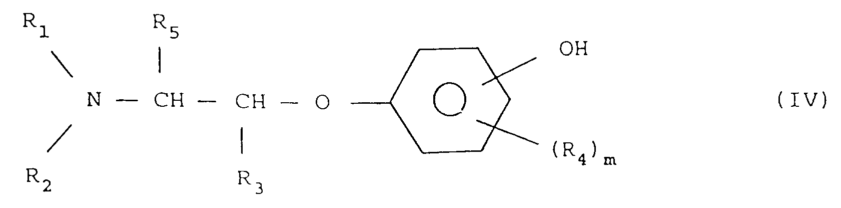 bildet, welches bei einer Umsetzung mit der Verbindung der allgemeinen Formel (V):
G-(CR'R'')ₙ-Y (V)
worin n, R', R'' und Y wie in Anspruch 1 definiert sind und G = Cl, Br, I, eine Methansulfonatester-Gruppe oder eine p-Toluolsulfonatester-Gruppe ist, in einem dipolaren protischen oder aprotischen Lösungsmittel in Gegenwart einer Base, bei einer Temperatur zwischen etwa 25°C und dem Siedepunkt der Reaktionsmischung eine Verbindung der allgemeinen Formel (I) ergibt; sowie gegebenenfalls die Umsetzung der erhaltenen Verbindung mittels bekannter Verfahren in das entsprechende Salz oder den Metallkomplex.

2. Verfahren gemäß Anspruch 1 zur Herstellung der Verbindungen der allgemeinen Formel (I), bei dem die heterocyclische (C₃-C₈)-Gruppe oder die heterocyclische (C₂-C₇)-Gruppe NR₁R₂ ausgewählt ist aus Morpholinyl, Piperidinyl und Thiomorpholinyl, wobei diese Gruppen gegebenenfalls mit mindestens einer Gruppe, ausgewählt aus (C₁-C₄)-Alkyl und Halogen, substituiert sind.

3. Verfahren gemäß Anspruch 1 zur Herstellung der Verbindungen der allgemeinen Formel (I), bei dem die heterocyclische (C₃-C₈)-Gruppe oder die heterocyclische (C₂-C₇)-Gruppe NR₁R₂ ausgewählt ist aus Morpholinyl, 2,6-Dimethyl-morpholinyl, Piperidinyl und 2,6-Dimethyl-piperidinyl.

4. Verfahren gemäß irgendeinem der Ansprüche 1 bis 3 zur Herstellung der Verbindungen der allgemeinen Formel (I), bei dem Y ausgewählt ist aus Pyridyl, Pyrimidinyl, Thienyl, Thiazolyl, Oxazolyl, Isoxazolyl, Cyclopropyl, Cyclobutyl, Cyclopentyl, Cyclohexyl, 1-Methyl-2,2-dichloro-cyclopropyl, Trimethylsilyl, tert. -Butyl-dimethylsilyl und Dimethylphenylsilyl.

5. Verfahren gemäß Anspruch 1 zur Herstellung der Verbindungen der allgemeinen Formel (I), d.h., 4-{3-[4-(Trimethylsilylmethoxy)-phenyl]-2-methyl-3-oxapropyl}-2,6-dimethylmorpholin; 4-{3-[3-(2-(4,6-Dichloro)-pyridyloxy)-phenyl]-2-methyl-3-oxapropyl}-2,6-dimethyl-morpholin; 4-{3-[3-(2,2-Dichloro-1-methyl-cyclopropylmethoxy)phenyl]-2-metyhl-3-oxapropyl}-2,6-dimethyl-morpholin; 4-[3-(5-Trifluoromethylpyridyloxy-2-yl)phenyl-2-methyl-3-oxapropyl]-2,6-dimethyl-morpholin; 4-[3-(3-Chloro-5-trifluoromethylpyridyloxy-2-yl)phenyl-2-methyl-3-oxapropyl]-2,6-dimethyl-morpholin; 4-[4-(Dimethylphenylsilylmethoxy)-phenyl-2-methyl-3-oxapropyl]-2,6-dimethyl-morpholin; 4-[3-(Dimethylphenylsilylmethoxy)-phenyl-2-methyl-3-oxapropyl]-2,6-dimethyl-morpholin; 4-{3-[1-(Trimethylsilyl)ethoxy]phenyl-2-methyl-3-oxapropyl}-2,6-dimethyl-morpholin; 4-{4-[1-(Trimethylsilyl)-ethoxy]phenyl-2-methyl-3-oxapropyl}-2,6-dimethyl-morpholin; oder 4-{3-[1-(Trimethylsilyl)ethoxy}phenyl-2-methyl-3-oxapropyl]-2,6-dimethyl-morpholin.

6. Fungizide Zusammensetzungen, welche mindestens eine der Verbindungen der allgemeinen Formel (I), welche gemäß irgendeinem der Ansprüche 1 bis 5 hergestellt wurden, zusammen mit einem Lösungsmittel und/oder einem Verdünnungsmittel enthalten.

7. Verwendung der Verbindungen der allgemeinen Formel (I) hergestellt gemäß irgendeinem der Ansprüche 1 bis 5 als Wirkstoffe zur Hemmung des Wachstums von Pilzen, welche für nützliche Kulturpflanzen pathogen sind, insbesondere von Pilzen, welche zur Art Erysiphe und Helminthosporium gehören.

## Revendications (Revendications pour l'(les) Etat(s) contractant(s) suivant(s): AT, BE, CH, LI, DE, DK, FR, GB, GR, IT, NL, SE)

1. Composés répondant à la formule générale (I) : 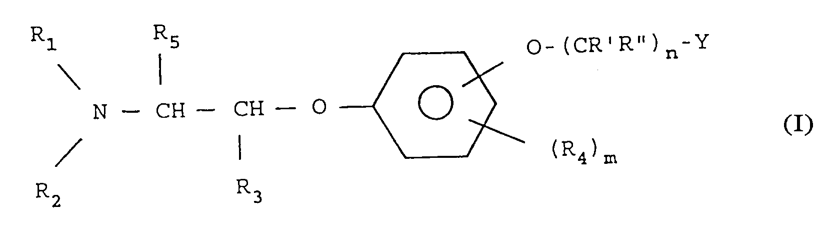 dans laquelle :
R₁ et R₂ conjointement liés à l'atome d'azote, représentent un groupe hétérocyclique en C₃-C₈ ou un groupe hétérocyclique en C₂-C₇ contenant un second hétéroatome choisi parmi O et S, lesdits groupes hétérocycliques étant éventuellement porteurs d'au moins un substituant choisi parmi des groupes alkyle en C₁-C₄ et des atomes d'halogène;
R₃ et R₅ représentent indépendamment un atome d'hydrogène ou un groupe alkyle en C₁-C₃;
R₄ représente un atome d'halogène, un groupe alkyle en C₁-C₃ ou halogénoalkyle en C₁-C₃;
m est un nombre entier compris entre 0 et 4;
R' et R'' représentent indépendamment un atome d'hydrogène ou un groupe alkyle en C₁-C₃;
n est un nombre entier compris entre 0 et 3; et
Y représente un groupe cycloalkyle en C₃-C₆ ou un groupe hétérocyclique à 5 ou 6 chaînons, lesdits groupes étant éventuellement porteurs d'au moins un substituant choisi parmi les groupes alkyle en C₁-C₄, alcoxy en C₁-C₃, halogénoalcoxy en C₁-C₃, halogénoalkyle et des atomes d'halogène; ou représente un groupe répondant à la formule 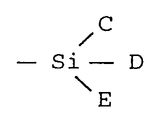 dans laquelle C, D et E représentent indépendamment un atome d'hydrogène, un groupe alkyle en C₁-C₄, halogénoalkyle en C₁-C₄, alcoxy en C₁-C₄, halogénoalcoxy en C₁-C₄, aryle en C₆-C₁₀ ou halogénoaryle en C₆-C₁₀,
ainsi que des composés énantiomères, diastéréoisomères, des sels de métaux et des complexes de ceux-ci.

2. Composés conformes à la revendication 1, dans lesquels ledit groupe hétérocyclique en C₃-C₈ ou ledit groupe hétérocyclique en C₂-C₇ du radical NR₁R₂ est choisi parmi les groupes morpholino, pipéridino et thiomorpholino, lesdits groupes étant éventuellement porteurs d'au moins un substituant choisi parmi des groupes alkyle en C₁-C₄ et des atomes d'halogène.

3. Composés conformes à la revendication 1, dans lesquels ledit groupe hétérocyclique en C₃-C₈ ou ledit groupe hétérocyclique en C₂-C₇ du radical NR₁R₂ est choisi parmi les groupes morpholino, 2,6-diméthyl-morpholino, pipéridino et 2,6 diméthylpipéridino.

4. Composé conforme à une quelconque des revendications 1 à 3, dans lequel Y est choisi parmi les groupes pyridyle, pyrimidinyle, thiényle, thiazolyle, oxazolyle, isoxazolyle, cyclopropyle, cyclobutyle, cyclopentyle, cyclohexyle, 1-méthyl-2,2-dichloro-cyclopropyle, triméthylsilyle, tert-butyldiméthylsilyle et diméthylphénylsilyle.

5. Composé conforme à la revendication 1, c'est à dire le 4-{3-[4-(triméthylsilylméthoxy)-phényl]-2-méthyl-3-oxapropyl)-2,6-diméthyl-morpholine;4-{3-[3-(2-(4,6-dichloro)-pyridyloxy)-phényl]-2-méthyl-3-oxapropyl}-2,6-diméthyl-morpholine; 4-{3-[3-(2,2-dichloro-1-méthyl-cyclopropylméthoxy)-phényl]-2-méthyl-3-oxapropyl}-2,6-diméthyl-morpholine; 4-[3-(5-trifluorométhylpyridyloxy-2-yl)-phényl-2-méthyl-3-oxapropyl]-2,6-diméthyl-morpholine; 4-[3-(3-chloro-5-trifluorométhylpyridyloxy-2-yl)-phényl-2-méthyl-3-oxapropyl]-2,6-diméthyl-morpholine; 4-[4-(diméthylphénylsilylméthoxy)-phényl-2-méthyl-3-oxapropyl]-2,6-diméthyl-morpholine; 4-[3-(diméthylphénylsilylméthoxy)-phényl-2-méthyl-3-oxapropyl]-2,6-diméthyl-morpholine; 4-{3-[1-(triméthylsilyl)éthoxy]-phényl-2-méthyl-3-oxapropyl}-2,6-diméthyl-morpholine; 4-{4-[1-(triméthylsilyl)éthoxy]-phényl-2-méthyl-3-oxapropyl}-2,6-diméthyl-morpholine; ou 4-{3-[1-(triméthylsilyl)éthoxy]-phényl-2-méthyl-3-oxapropyl}-2,6-diméthyl-morpholine.

6. Procédé pour la préparation des composés définis dans une quelconque des revendications précédentes, comprenant l'hydrogénation d'un composé de type carbonyle répondant à la formule générale (II) : 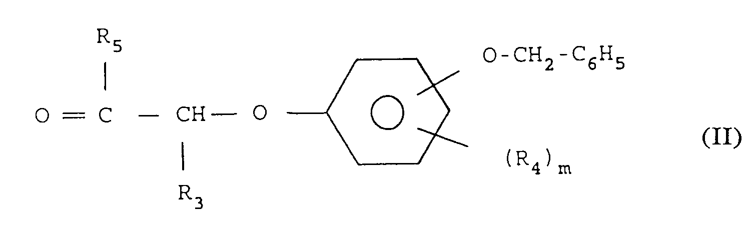 dans laquelle R₃, R₄, R₅ et m ont la même signification que dans la revendication 1; après l'addition d'une amine de formule générale (III) : 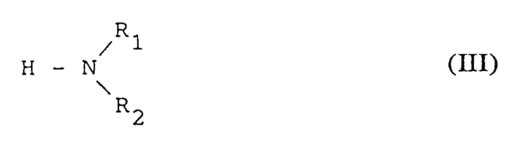 dans laquelle R₁ et R₂ ont la même signification que dans la revendication 1; en présence d'un catalyseur d'hydrogénation et à une température comprise entre 0 et 60°C environ et une pression d'hydrogène comprise entre 0,1 et 1,1 MPa environ (comprise entre 1 et 10 atmosphères environ), résultant à la formation d'une amine répondant à la formule générale (IV) : 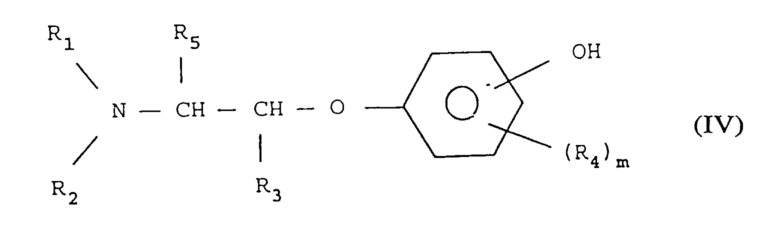 qui, après réaction avec le composé répondant à la formule générale (V) :
G-(CR'R'')ₙ-Y (V)
dans laquelle n, R', R'' et Y ont la même signification que dans la revendication 1 et G = Cl, Br, I, un groupe méthane-sulfonyloxy ou p-toluène-sulfonyloxy; en présence d'une base en solution dans un solvant dipolaire protique ou aprotique et à une température comprise entre environ 25°C et la température d'ébullition du mélange réactionnel, aboutit au composé répondant à la formule générale (I); et éventuellement la conversion du composé obtenu en son sel ou son complexe métallique correspondant, par des moyens classiques.

7. Compositions fongicides, contenant au moins un des composés conforme à une quelconque des revendications 1 à 5, en solution dans un solvant et/ou un diluant.

8. Utilisation des composés conformes à une quelconque des revendications 1 à 5, comme agent inhibiteur de la croissance de champignons pathogènes dans les cultures de plantes utiles, en particulier de champignons appartenant aux genres Erysiphe et Helminthosporium.

## Revendications (Revendications pour l'(les) Etat(s) contractant(s) suivant(s): ES)

1. Procédé pour la préparation de composés répondant à la formule générale (I) : 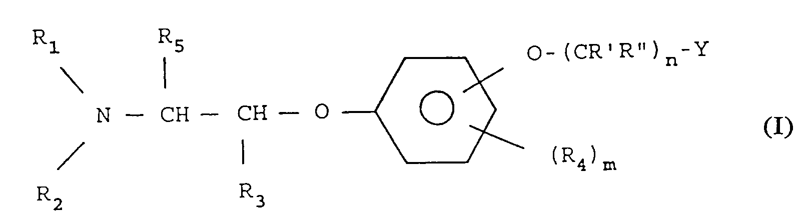 dans laquelle :
R₁ et R₂ conjointement liés à l'atome d'azote, représentent un groupe hétérocyclique en C₃-C₈ ou un groupe hétérocyclique en C₂-C₇ contenant un second hétéroatome choisi parmi O et S, lesdits groupes hétérocycliques étant éventuellement porteurs d'au moins un substituant choisi parmi des groupes alkyle en C₁-C₄ et des atomes d'halogène;
R₃ et R₅ représentent indépendamment un atome d'hydrogène ou un groupe alkyle en C₁-C₃;
R₄ représente un atome d'halogène, un groupe alkyle en C₁-C₃ ou halogénoalkyle en C₁-C₃;
m est un nombre entier compris entre 0 et 4;
R' et R'' représentent indépendamment un atome d'hydrogène ou un groupe alkyle en C₁-C₃;
n est un nombre entier compris entre 0 et 3; et
Y représente un groupe cycloalkyle en C₃-C₆ ou un groupe hétérocyclique à 5 ou 6 chaînons, lesdits groupes étant éventuellement porteurs d'au moins un substituant choisi parmi les groupes alkyle en C₁-C₄, alcoxy en C₁-C₃, halogénoalcoxy en C₁-C₃, halogénoalkyle et des atomes d'halogène; ou représente un groupe répondant à la formule 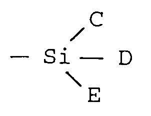 dans laquelle C, D et E représentent indépendamment un atome d'hydrogène, un groupe alkyle en C₁-C₄, halogénoalkyle en C₁-C₄, alcoxy en C₁-C₄, halogénoalcoxy en C₁-C₄, aryle en C₆-C₁₀ ou halogénoaryle en C₆-C₁₀,
ainsi que des composés énantiomères, diastéréoisomères, des sels de métaux et des complexes de ceux-ci, comprenant l'hydrogénation d'un composé de type carbonyle répondant à la formule générale (II) : 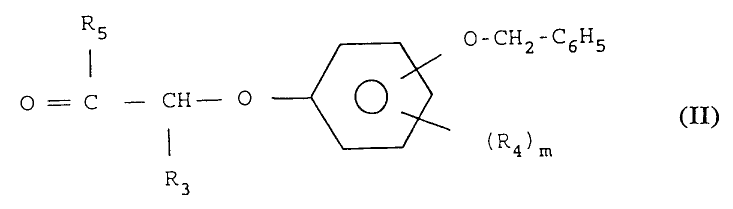 dans laquelle R₃, R₄, R₅ et mont la même signification que dans la revendication 1; après l'addition d'une amine de formule générale (III) : 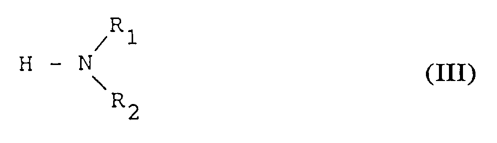 dans laquelle R₁ et R₂ ont la même signification que ci-dessus; en présence d'un catalyseur d'hydrogénation et à une température comprise entre 0 et 60°C environ et une pression d'hydrogène comprise entre 0,1 et 1,1 MPa environ (comprise entre 1 et 10 atmosphères environ), résultant à la formation d'une amine répondant à la formule générale (IV) : 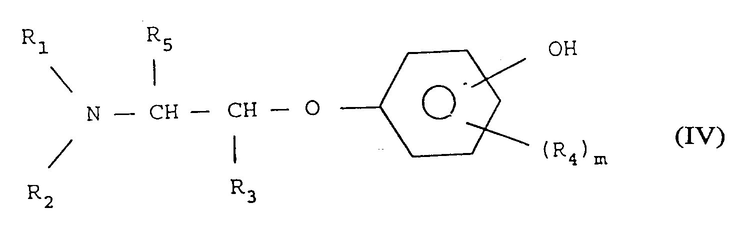 qui, après réaction avec le composé répondant à la formule générale (V) :
G-(CR'R'')ₙ-Y (V)
dans laquelle n, R', R'' et Y ont la même signification que dans la revendication 1 et G = Cl, Br, I, un groupe méthane-sulfonyloxy ou p-toluène-sulfonyloxy; en présence d'une base en solution dans un solvant dipolaire protique ou aprotique et à une température comprise entre environ 25°C et la température d'ébullition du mélange réactionnel, aboutit au composé répondant à la formule générale (I); et éventuellement la conversion du composé obtenu en son sel ou son complexe métallique correspondant, par des moyens classiques.

2. Procédé conforme à la revendication 1 pour la préparation de composés répondant à la formule générale (I) , dans lequel ledit groupe hétérocyclique en C₃-C₈ ou ledit groupe hétérocyclique en C₂-C₇ du radical NR₁R₂ est choisi parmi les groupes morpholino, pipéridino et thiomorpholino, lesdits groupes étant éventuellement porteurs d'au moins un substituant choisi parmi des groupes alkyle en C₁-C₄ et des atomes d'halogène.

3. Procédé conforme à la revendication 1 pour la préparation de composés répondant à la formule générale (I), dans lequel ledit groupe hétérocyclique en C₃-C₈ ou ledit groupe hétérocyclique en C₂-C₇ du radical NR₁R₂ est choisi parmi les groupes morpholino, 2,6-diméthylmorpholino, pipéridino et 2,6 diméthylpipéridino.

4. Procédé conforme à une quelconque des revendications 1 à 3 pour la préparation de composés répondant à la formule générale (I), dans lesquels Y est choisi parmi les groupes pyridyle, pyrimidinyle, thiényle, thiazolyle, oxazolyle, isoxazolyle, cyclopropyle, cyclobutyle, cyclopentyle, cyclohexyle, 1-méthyl-2,2-dichloro-cyclopropyle, triméthylsilyle, tert-butyldiméthylsilyle et diméthylphénylsilyle.

5. Procédé conforme à la revendication 1 pour la préparation de composés répondant à la formule générale (I), c'est à dire le 4-{3-[4-(triméthylsilylméthoxy)-phényl]-2-méthyl-3-oxapropyl}-2,6-diméthyl-morpholine;4-{3-[3-(2-(4,6-dichloro)-pyridyloxy)-phényl]-2-méthyl-3-oxapropyl}-2,6-diméthyl-morpholine; 4-{3-[3-(2,2-dichloro-1-méthylcyclopropylméthoxy)-phényl]-2-méthyl-3-oxapropyl}-2,6-diméthyl-morpholine; 4-[3-(5-trifluorométhylpyridyloxy-2-yl)-phényl-2-méthyl-3-oxapropyl]-2,6-diméthyl-morpholine; 4-[3-(3-chloro-5-trifluorométhylpyridyloxy-2-yl)-phényl-2-méthyl-3-oxapropyl]-2,6-diméthyl-morpholine; 4-[4-(diméthylphénylsilylméthoxy)-phényl-2-méthyl-3-oxapropyl]-2,6-diméthyl-morpholine; 4-[3-(diméthylphénylsilylméthoxy)-phényl-2-méthyl-3-oxapropyl]-2,6-diméthyl-morpholine; 4-{3-[1-(triméthylsilyl)éthoxy]-phényl-2-méthyl-3-oxapropyl}-2,6-diméthyl-morpholine; 4-{4-[1-(triméthylsilyl)éthoxy]-phényl-2-méthyl-3-oxapropyl}-2,6-diméthyl-morpholine; ou 4-{3-[1-(triméthylsilyl)éthoxy]-phényl-2-méthyl-3-oxapropyl}-2,6-diméthyl-morpholine.

6. Compositions fongicides, contenant au moins un des composés répondant à la formule générale (I) préparés conformément à une quelconque des revendications 1 à 5, en solution dans un solvant et/ou un diluant.

7. Utilisation des composés répondant à la formule générale (I) préparés conformément à une quelconque des revendications 1 à 5, comme agent inhibiteur de la croissance de champignons pathogènes dans les cultures de plantes utiles, en particulier de champignons appartenant aux genres Erysiphe et Helminthosporium.
